# EUROPEAN PATENT SPECIFICATION

(11) **EP 1 311 297 B1**
(45) Date of publication and mention of the grant of the patent: **26.07.2006**
(21) Application number: 01960783.7
(22) Date of filing: 31.07.2001
(51) Int. Cl.: A61K 48/00, A61K 39/395, C07K 14/575

(54) **A METHOD FOR REDUCING STRESS-INDUCED OVERPRODUCTION OF NEUROPEPTIDE Y IN AN INDIVIDUAL**
METHODE ZUR STESSINDUZIERTEN NEUROPEPTID-Y-ÜBERPRODUKTION IN EINEM INDIVIDUUM
PROCEDE POUR REDUIRE LA SURPRODUCTION DE NEUROPEPTIDE Y CHEZ UNE PERSONNE

(30) Priority: 25.08.2000 US 645590
(43) Date of publication of application: 21.05.2003
(73) Proprietor: Phase One Pharma Oy Ltd., 20520 Turku (FI)
(72) Inventor: KOULU, Markku, FIN-20700 Turku (FI); KARVONEN, Matti, FIN-20520 Turku (FI); PESONEN, Ullamari, FIN-20900 Turku (FI); UUSITUPA, Matti, FIN-70260 Kuopio (FI); KALLIO, Jaana, FIN-20540 Turku (FI)
(74) Representative: Öhman, Ann-Marie
(86) International application number: PCT/FI2001/000687
(87) International publication number: WO 2002/015941

(56) References cited:
- WO-A1-96/34877
- WO-A1-99/32518
- MARGARET J. MORRIS ET AL.: 'Region-specific neuropeptide Y overflows at rest and during sympathetic activation in humans' HYPERTENSION vol. 29, 1997, pages 137 - 143, XP002906855
- JAN M. LUNDBERG ET AL.: 'Co-release of neuropeptide Y and catecholamines during physical exercise in man' BIOCHEMICAL AND BIOPHYSICAL RESEARCH COMMUNICATIONS vol. 133, no. 1, November 1985, pages 30 - 36, XP002906856
- JAANA KALLIO ET AL.: 'Altered intracellular processing and release of neuropeptide Y due to leucine 7 to proline 7 polymorphism in the signal peptide of preproneuropeptide Y in humans' FASEB J. vol. 15, no. 7, May 2001, pages 1242 - 1244, XP002906857

## Description

### FIELD OF THE INVENTION

This invention concerns a method for reducing stress-induced overproduction of neuropeptide Y (NPY) in an individual.

### BACKGROUND OF THE INVENTION

Neuropeptide Y (NPY) is the most abundant neuropeptide and an important neurotransmitter in the human central and peripheral nervous systems. Trough five cloned widely expressed receptors (Y1-Y5) it regulates e.g. food intake, vasoconstriction, hormone release, kidney function, lipolysis and even angiogenesis¹⁻⁹. Exogenously given NPY is a potent vasoconstrictor and modifies the vasoconstriction achieved by norepinephrine (NE) ¹⁰⁻¹². NPY is co-stored and co-released with NE in all sympathetic nerve terminals innervating different organs ¹³⁻¹⁶ . In plasma, NPY concentration is considered to reflect the level of sympathetic activation in rest and during exercise¹⁷⁻²⁰.

A recent study found a Leucine 7 to Proline 7 (Leu7Pro) polymorphism in the signal peptide of NPY and indicated significant association of this polymorphism to high serum total and low-density (LDL) cholesterol levels, especially in obese subjects²¹). The carrier frequency for this polymorphism is 6 to 13% in studied European populations²¹. The same polymorphism is also associated with higher serum triglyseride levels in preschool-aged children²² and with accelerated atherosclerosis in middle-aged men and type II diabetic patients as well as with early development of diabetic retinopathy in these patients²³. Baby boys born with this polymorphism are heavier at birth²², but later in life the polymorphism does not associate with body mass index (BMI) or absolute food intake or food preferences²¹.

The mechanisms of how the mutated preproNPY mediates the important blood lipid and vascular changes are not known, but may include altered secretion of NPY from cells to circulation or locally to tissues. The newly formed preproNPY is a 96-amino acid (aa) peptide, which has a 28-aa signal peptide in the N-terminal end and a 32-aa C-ponderal peptide (C-pon) in the other end. The signal peptide guides the peptide into endoplasmic reticulum (ER). In the ER, the signal peptide is cleaved off and the remaining proNPY undergoes further modification by several enzymes into mature 36-residue amidated NPY, which is stored in secretory vesicles²⁴. It is proposed that the secondary and tertiary structure of the signal peptide may be altered dramatically by the Leu7Pro -switch as proline easily forms brakes and kinks in alpha-helical structures^{25,26}. Therefore, the found polymorphism may modify the formation, storage or release kinetics of the mature NPY.

To study the functional role of this polymorphism, sympathetic responses including NPY excretion were induced by strenuous physical exercise in Leu7Pro and Leu7Leu genotype groups of healthy subjects. To further evaluate the functional consequences of this polymorphism, endothelial cells, which are known to contain and secrete NPY⁹ were used for immunocytochemical studies. Human umbilical vein endothelial cells (HUVEC) were isolated, genotyped and studied by confocal microscopy to determine the patterns of NPY- and proNPY-ir in Leu7Pro compared to Leu7Leu cells.

### SUMMARY OF THE INVENTION

This invention concerns the use of a substance for the manufacture of a pharmaceutical composition for use in a method for reducing stress-induced overproduction of neuropeptide Y (NPY) in an individual, wherein the stress-induced overproduction of NPY is caused by a polymorphism comprising the substitution of the position 7 leucine for proline in the signal peptide part of the human preproNPY, wherein
- said method is aimed to modulate an overactive NPY system in said individual, or
- said individual is subj ected to a method aimed to reduce or prevent expression of the mutated allele causing said polymorphism, and said substance is
   - a Y1-receptor antagonist selected from the group consisting of BIBO 3304; BIBP 3226; GR231118; and a 1,3-disubstituted benzazepine,
   - a Y2-receptor antagonist which is BIIE0246,
   - a Y5-receptor antagonist selected from the group consisting of L-152,804 and CGP71683A,
   - an NPY-antibody,
   - an antisense oligonucleotide,
   - a peptide nucleic acid (PNA), or
   - a ribozyme.

According to a further aspect, this invention concerns a method for diagnosing a person's susceptibility for having a risk for stress-induced overproduction of neuropeptide Y (NPY), said method comprising determining in vitro whether said subject has a polymorphism in the signal peptide part of the human preproNPY, said polymorphism comprising the subsitution of the position 7 leucine for proline in the signal peptide part of said preproNPY, said polymorphism being indicative of a risk for stress-induced overproduction of NPY.

### BRIEF DESCRIPTION OF THE DRAWINGS

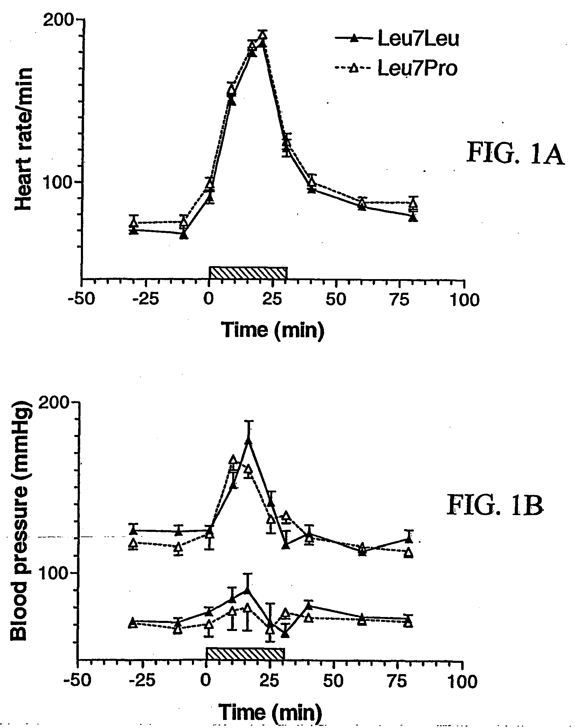

Figures 1A and 1B show the heart rate and blood pressure. Heart rate (Figure 1A) was significantly higher (p < 0.05) in subjects with Leu7Pro genotype (open triangles) than in subjects with Leu7Leu genotype (solid triangles). No differences were detected in systolic (upper curves) or diastolic blood pressure (lower curves) between the groups (Figure 1B). The exercise-time was 30 min (hatched area).

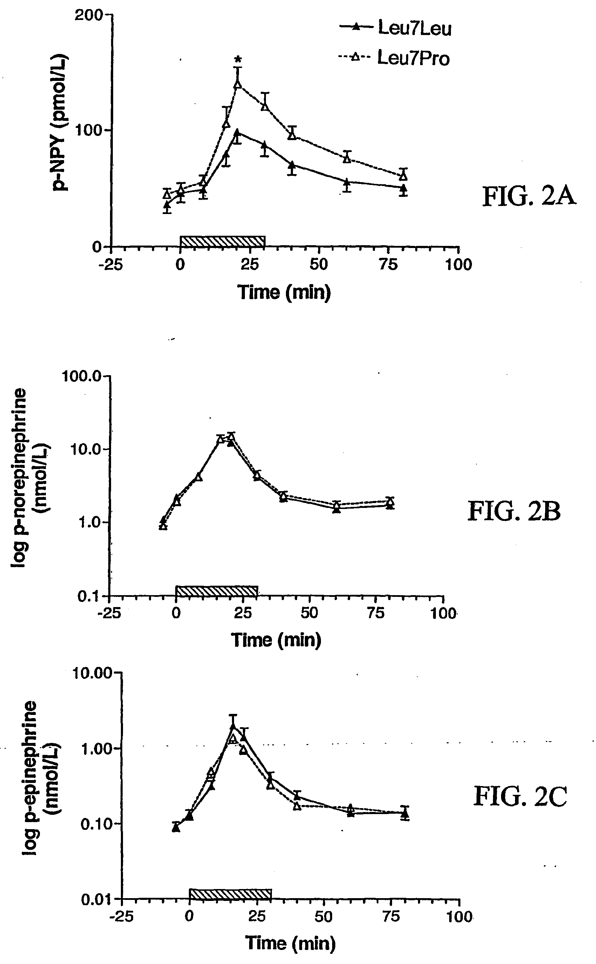

Figures 2A, 2B and 2C show the plasma concentration of NPY, norepinephrine and epinephrine. Overall and the mean plasma NPY concentrations (Figure 2A) at 20 min (*) were significantly higher (p < 0.05) in subjects with Leu7Pro genotype (open triangles) than in subjects with Leu7Leu genotype (solid triangles). Similar catecholamine concentrations (Figures 2B and 2C) in plasma were detected in the two study groups. The exercise-time was 30 min (hatched area).

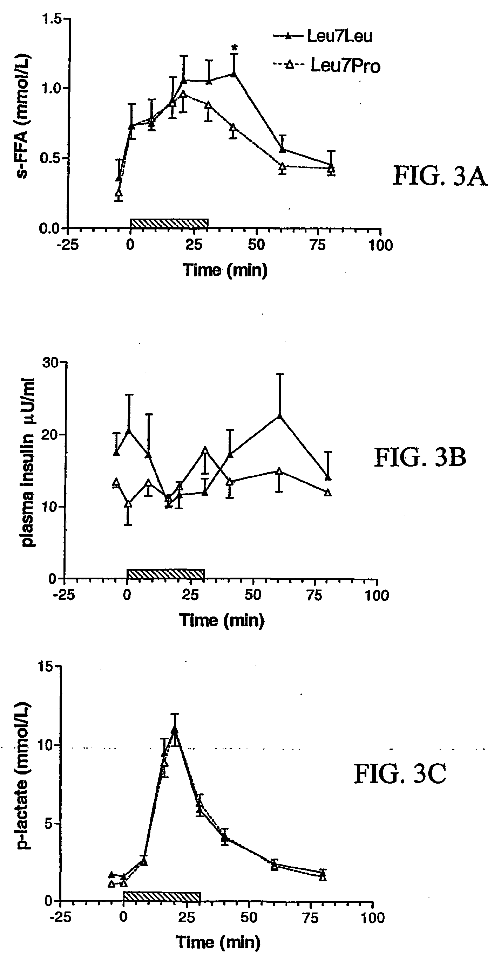

Figures 3A, 3B and 3C show the serum FFA, plasma insulin and plasma lactate concentrations. Overall and the mean serum FFA concentrations (Figure 3A) at 40 min (*) were significantly lower (p < 0.05) in subjects with Leu7Pro genotype (open triangles) than in subjects with Leu7Leu genotype (solid triangles). Different overall insulin concentrations (Figure 3B) were detected between the genotypes with significant differences (*) at 0min (p <0.05) and at 60 min. Lactate concentrations in plasma (Figure 3C) were similar in the two study groups. The exercise-time was 30 min (hatched area).

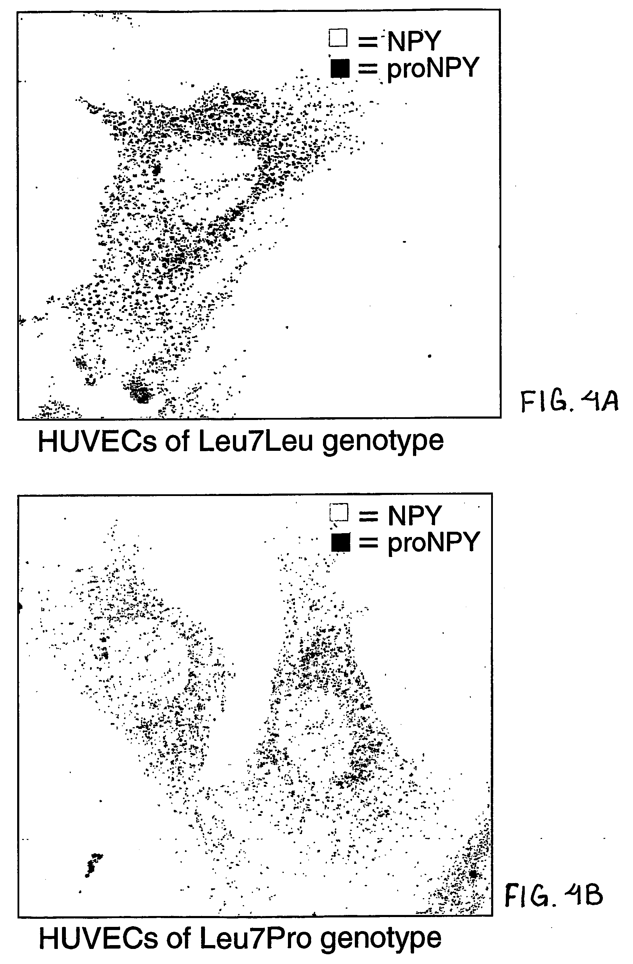

Figures 4A and 4B show printed pictures based on scanning confocal images of HWECs with Leu7Leu genotype (Figure 4A) and Leu7Pro genotype (Figure 4B), double-immunostained with rabbit polyclonal anti-human C-pon antibody and goat polyclonal anti-human NPY antibody. The images are summations of 8 mid-sections of permeabilized cells. Prominent overlapping staining of NPY and C-pon was seen in cells with Leu7Leu genotype (Figure 4A), indicating overwhelming presence of proNPY. NPY without C-pon was abundant in vesicle-like structures in HUVECs with Leu7Pro genotype (Figure 4B), these cells had also some proNPY staining.

### DETAILED DESCRIPTION OF THE INVENTION

The study presented below shows that a Leu7Pro polymorphism in the signal peptide part of the human preproNPY causes a stress-induced overproduction of NPY in these individuals during exercise. Because the overproduction of NPY in turn can give rise to undesirable conditions and many diseases, we believe that it is essential to decrease the production of NPY to a normal level.

The term "overproduction" shall in this document be understood to cover excessive expression, excessive release, or increased intracellular formation, distribution or storage.

Although this study shows that the stress-induced overproduction of NPY is related to Leu7Pro polymorphism in the signal peptide part of the human preproNPY, we do not exclude that such stress-induced overproduction also could be caused by other factors. We believe that it is essential to decrease overproduction of NPY irrespective of the mechanism behind it.

The International Patent Publication WO 99/32518 shows the relationship between Leu7Pro polymorphism and increased total cholesterol and increased LDL-cholesterol in serum. Figures 1a to 1c show the location of the Leu7Pro polymorphism in the NPY-gene.

Thus, according to one preferred embodiment, the overproduction of NPY is counteracted by administering an antagonist to said individual.

The antagonist can be one aimed to decrease the expression of the NPY gene.

As examples can be mentioned Y1-, Y2- and Y5-receptor antagonists. As examples of Y1-receptor antagonists can be mentioned BIBO 3304 (Br J Pharmacol 125, 549-55, 1998); BIBP 3226 (Regul Pept 25, 377-82, 1998); GR231118 (Bur J Pharmacol 349, 97-105, 1998); and 1,3-disubstituted benzazepines (Bioorg Med Chem 1703-14, 1999). As example of Y2-receptor antagonists can be mentioned BIIE0246 (Eur J Pharmacol 396, R1-3, 2000 Br J Pharmacol 129, 1075-1088, 2000). As examples of Y5-receptor antagonists can be mentioned L-152,804 (Biochem Biophys Res Commun 272, 169-173, 2000) and CGP71683A (Can J Physiol Pharmacol 78, 116-25, 2000).

According to a further alternative, the antagonist can be an NPY antibody. Such an antibody can be either bound to tissue or a soluble antibody in free form in serum. In the latter case, such an antibody can catch the NPY in serum and form a complex with the same. Thereby the binding of NPY to the NPY receptors is prevented.

In the alternatives mentioned above, the overproduction of NPY can be caused by a polymorphism comprising the substitution of the position 7 leucine for proline in the signal peptide part of the human preproNPY or by any other reason.

According to another embodiment, in case the overproduction of NPY is caused by a polymorphism comprising the substitution of the position 7 leucine for proline in the signal peptide part of the human preproNPY, the individual is subjected to a method aimed to reduce or prevent expression of the mutated allele causing said polymorphism.

Such a method can be a specific gene therapy aimed to repair the mutated allele, for example by use of an antisense oligonucleotide, a peptide nucleid acid (PNA), or ribozyme.

The antisense therapy refers to methods designed to impair translation through direct interactions with target messenger RNA (mRNA). This can be accomplished by applying a targeted oligonucleotide, which forms Watson-Crick base pairs with the messenger RNA whose function is to be disrupted.

The inhibition of gene expression by antisense oligonucleotide depends on the ability of an antisense oligonucleotide to bind a complementary mRNA sequence and prevent the translation of the mRNA. It is possible to correct a single mutant base in a gene by using an oligonucleotide based strategy (Giles et al., 1995; Schwab et al., 1994; Yoon et al., 1996). A short, 7 or 8 bases, oligonucleotide is enough to posses an antisense activity and specificity, which depends greatly on the flanking sequences of the target RNA. Binding should be enough to promote stable binding and RNase H - mediated cleavage.

We are counteracting the influence of the mutated NPY gene by using a short, allele specific oligonucleotide, which includes the sequence of mutated part: ...cga ct/cg ggg...(mutated base marked on bold). This can be accomplished by using oligonucleotides of various lengths, but all recognizing the mutated base sequence. According to the predicted secondary structure of the preproNPY mRNAs (Scheme 1 and 2), the best target sequence is between -9 and +2 bases around the mutation i.e. sequence targeting to 3'-ac aag cga ctg g-5'. This sequence contains 'bulbs' which are known to enhance the binding of oligonucleotide to the target mRNA.

It is possible to use unmodified oligonucleotides, but to increase their stability, nuclease resistance, and penetration to the nucleus, several modifications of oligonucleotide can be used. A relatively large number of modified pyrimidines have been synthesized, mainly C-2, C-4, C-5, and C-6 sites, and incorporated into nucleotides. Also purine analogs can be synthesized and incorporated into oligonucleotides. The 2' position of the sugar moiety, pentofuranose ring, is substituted with methoxy, propoxy, O-alkoxy or methoxyethoxy groups. A new backbone for oligonucleotides that replace the phosphate or the sugar-phosphate unit has been made, like C-5 propynylpyrimidine-modified phosphothioate oligonucleotides. Also chimeric oligonucleotides with 5'- and 3'-ends are modified with internucleotide linkages, like methylphosphorothioate, phosphodiester, or methylphosphonate can be used. A relatively new technique is conformationally restricted LNA (locked nucleic acid) oligonucleotides and peptide nucleic acids. Bioengineered ribozymes are structurally different, but their specificity also relay on the recognition of the targeted mRNA sequence.

Gene replacement or gene switching techniques inactivate the mutated gene sequence and introduce a corrected one. This can be accomplished by transfecting exogenous gene with normal coding sequence and blocking mutant coding sequence with antisense oligonucleotide. Also a technique with only introducing a corrected normal sequence without disrupting the mutated sequence could be use. This could be used in heterozygous cells i.e. cell carrying one normal allele and one mutated allele resulting in an overexpression of normal alleles. Also homozygous mutant cells could be treated resulting in a dominant positive -effect i.e. the normal allele is expressed in higher degree than the mutant allele.

The ribozyme technology is described for example in the following publications: Ribozyme protocols: Turner, Philip C (editor). Humana Press, ISBN 0-89603-389-9, 512 pp. 1997; Rossi JJ. Ribozymes, genomics and therapeutics. Chem Biol 6, R33-7, 1999; and Ellington AD, Robertson MP, Bull J. Ribozymes in wonderland. Science 276, 546-7, 1997.

The PNA technology is described in Ray A, Norden, B. Peptide nucleic acid (PNA): its medical and biotechnical applications and promise for the future. FASEB J 14, 1041-1066, 2000.

The invention will be illuminated by the following non-restrictive Experimental Section.

### EXPERIMENTAL SECTION

The rather common Leu7Pro polymorphism in the signal peptide of neuropeptide Y (NPY) has previously been shown to be associated with high blood lipid concentrations and also to accelerated atherosclerosis independently from blood lipid values. We studied the effects of the Leu7Pro genotype on NPY secretion and processing in humans and in isolated human endothelial cells expressing endogenously NPY. NPY secretion and other symphatetic responses were stimulated in nine subjects having the Leu7Pro genotype and in nine matched Leu7Leu genotype controls by strenuous cycle-ergometer exercise. Subjects with the Leu7Pro genotype had 42 % higher maximal increases in the plasma NPY concentrations than subjects with the Leu7Leu genotype. They had also significantly higher heart rate and lower exercise-induced free fatty acid concentrations, which may be secondary changes to increased NPY levels. Furthermore, clearly more NPY-immunoreactivity was observed in human endothelial cells with the Leu7Pro genotype as verified by confocal microscopy. In conclusion, the present study provides the first direct evidence of functional consequences of the Leu7Pro polymorphism leading to more efficient production and release of NPY. Thus, overproduction of NPY may lead to atherosclerosis and unfavourable changes in plasma lipids. The present observations may provide new strategies for prevention and treatment of these diseases and other disorders caused by overproduction of NPY.

### Methods

### Study subjects

The joint Ethics Committee of Turku University and Turku University Central Hospital approved all parts of the study. Written informed consent was obtained from each subject for genotyping and for participation in maximal oxygen consumption (Vo₂ₘₐₓ) determination and 80% Vo₂ₘₐₓ bicycle exercise test (workload 80% of the determined Vo₂ₘₐₓ corresponding 100% workload). Participation for the genotype screening was offered to non-selected subjects over 18 years of age with no diseases as ascertained by questioning. Nine subjects having the Leu7Pro genotype (Table 1) and nine pair-matched controls (matched for age, sex and BMI) with the Leu7Leu genotype (Table 1) were selected for Vo₂ₘₐₓ determination and the 80% Vo₂ₘₐₓ cycle-ergometer exercise test based on the former genotyping. To exclude non-healthy subjects, detailed medical history (including diseases, medication, smoking, trauma, alcohol consumption) was taken and a physical examination (including ECG, auscultation, blood pressure measurement, thyroid palpation, screening for clinical signs of infection) conducted before the subjects entered the study. Also, laboratory measurements (blood hemoglobin, total cholesterol, LDL-cholesterol, glucose, free fatty acid and alanine amino transferase concentration, leucocyte count, and erythrocyte sedimentation rate) were done to exclude sick subjects. Body fat was determined by skin-fold measurement²⁷.

### Genotyping

For genotyping, blood samples (10ml) were drawn from an antecubital vein and isolated HUVEs in culture were harvested. Blood leukocyte DNA and HUVEC DNA were extracted using DNA isolation kits Purugene and Capture Column Kit, respectively (Gentra Systems, Minneapolis, MN, USA) following the manufacturer's instructions. The genotype was determined using polymerase chain reaction (PCR) to amplify part of the preproNPY gene (237 bp). Thymidine-1128 to cytosine substitution results in the Leucine to Proline switch in position seven in preproNPY, which was_detected by digestion of the PCR product with Bsi E1 (New England Biolabs Inc., Beverly, MA, USA) and restriction fragment length polymorphism analysis (189bp and 48 bp fragments are produced by the mutant Pro7 allele).

### Study protocol

The 18 healthy study subjects were asked to refuse from any medication, alcohol -containing drinks or food for 48 hours and from any caffeine- containing drinks or food for 12 hours before the Vo₂ₘₐₓ measurement and before the 80% Vo₂ₘₐₓ bicycle exercise test. They were asked to eat lots of carbohydrate-rich food and to avoid strenuous physical exercise for two days preceding the tests. A standard light meal was offered 2 h before running the tests. The individual maximal oxygen uptake (Vo₂ₘₐₓ) was determined in the Paavo Nurmi Centre, Turku. The initial power output was 40 W for women and 60 W for men and was increased by 20 W and 30 W, respectively, every 2 min until exhaustion. Respiratory gases were analyzed (AMETEK S-3A1 O₂ and Beckman LB-2 CO₂ analyzer) and recorded every 15 seconds by an online real-time PC-based system. Respiratory exchange ratio (> 1.15) and oxygen uptake (Vo₂) uniformity were evaluated to ensure a true maximum effort. The highest Vo₂ was determined and the corresponding power level was considered as 100% Vo₂ₘₐₓ. In the 80% Vo₂ₘₐₓ exercise study, an intravenous cannula was inserted into an antecubital vein and the subjects were lying for 35 minutes (min) thereafter. Before starting to run the bicycle (at 0 min), they were sitting on the cycle-ergometer for 5 min and at 0 min the study subjects started to exercise with minimal (20 W) workload. The workload was increased by 20% VO₂ₘₐₓ steps in 2-min intervals and the 80% Vo₂ₘₐₓ workload was thus reached after 4 steps in 8 min. The study subjects then continued to exercise at this 80% Vo₂ₘₐₓ level for 12 min followed for 10 min cooling with 20% Vo₂ₘₐₓ workload. The study subjects were monitored (EKG and blood pressure) by Datex Engstrom AS/3-system (Datex Ohmeda, Oulu, Finland) for a 30-minute baseline period before the exercise, during the 30-min exercise and also 50 min in a sitting position after the exercise. Nine blood samples of 15 ml (for the measurement the of plasma NPY, epinephrine (E), norepinephrine, lactate, insulin and serum free fatty acids (FFA)), were collected and heart rate recorded at -5, 0, 8, 16, 20, 30, 40, 60 and 80 min. Heart rate was additionally recorded at -30 min. Blood pressure was measured at -30, -10, 0, 16, 25, 30, 40, 60 and 80 min.

### Analytical methods

Plasma NPY concentrations were determined using a commercial radioimmunoassay kit (EURIA-NPY, Euro-Diagnostica Inc., Malmö, Sweden). NE and E concentrations in plasma were determined using high performance liquid chromatography with electrochemical detection²⁸. This method had the intra- and interassay variances for both NE and E below 12%. FFA concentrations in serum were determined with NEFA-C Reagent set (Wako Chemicals GmbH, Neuss, Germany) and plasma lactate concentrations with an enzymatic UV-method (Roche Diagnostics GmbH, Mannheim, Germany) using Hitachi 917 Automatic Analyzer (Hitachi Ltd., Tokyo, Japan). The interassay variance was 0.6% for FFA (n=37) at 0.62 mmol/L and 1.6% for lactate at 2.10 mmol/L (n = 47). Plasma insulin concentrations were determined by radioimmunoassay kit INSIK-5 (DiaSorin s.r.1., Saluggia, Italy). Other laboratory measurements were done by standard methods.

### Statistical analysis

Baseline characteristics (Table 1) of study subjects were compared using unpaired two-tailed t tests. Chatecolamine (E, NE) values were log-transformed before further analysis. The means of each sequentially measured parameter between genotypes Leu7Pro and Leu7Leu were compared using repeated measures ANOVA for mixed models (Table 2). If the ANOVA revealed statistically significant genotype-by-time interaction (overall difference) the Fisher least significant difference multiple comparison procedure was used to test equality of group means at each time point. These tests were carried out as linear contrasts using the same statistical model. For correlation analysis, Pearson's correlation coefficients were calculated. All data are presented as mean ± SEM. Statistical analysis was performed with SAS software (Version 6.12, SAS Institute Inc., Cary, NC, USA).

### Cell culture and immunocytochemical detection of proNPY and NPY

HWECs were isolated from freshly delivered umbilical cords using collagenase type II (0.3 mg/ml) and type IV (0.3 mg/ml) enzymes (Sigma Chemical Co., St Louis, MO, USA) in PBS at 37 °C for 15 minutes. Detached cells were seeded into 0.2 % gelatin-coated (Sigma Chemical Co., St Louis, MO, USA) 25 cm² cell culture flasks. HWECs were grown in Medium-199 (Life Technologies Ltd., Paisley, Scotland) supplemented with 2.5 U/ml sodium heparin, 10 % heat inactivated FBS (Autogen Bioclear, Wiltshire, United Kingdom), 100 U/ml penicillin, 100 µg/ml streptomycin (BioWittaker, Walkersville, MD, USA), 2 mM L-glutamine (Life Technologies Ltd., Paisley, Scotland), 50 µg/ml gentamycin (BioWittaker, Walkersville, MD, USA) and 25 µg/ml ECGS (Endothelial Cell Growth Supplement) (Upstate Biotechnology, Lake Placid, NY, USA). Fresh media was added every other day and the cells were replated every 2-3 days. Experiments were performed with cells between passages 2 and 7. For immunocytochemistry, cells were plated on 0.2 % gelatin-coated glass coverslips at 1-2 x 10⁴ cells /cm² and were fixed after 24-48 h at room temperature for 20 min with 4% paraformaldehyde. The immunocytochemical reactions were performed at room temperature. Nonspecific binding was blocked by incubating the cells for 45 min with blocking buffer containing 0.2% Nonidet P40 (Calbiochem, Novabiochem Corp., La Jolla, CA, USA) as permeabilizing agent and 5% non-fat dry milk in 50 mM Tris-HCl, pH 7.6. The cells were double-stained by 45 min incubation with two primary antibodies, rabbit polyclonal anti-human C-pon antibody (1:200) (Affiniti Research Products Ltd., Mamhead, United Kingdom) and goat polyclonal anti-human NPY antibody (1:100) (Affiniti Research Products Ltd., Mamhead, United Kingdom) in the same buffer. After incubation the cells were rinsed three times with PBS, followed by 5 min blocking and two subsequent 30-min incubations with secondary antibodies FITC-conjugated anti-rabbit IgG (1:500) (Silenius Laboratories, Hawthorne, Australia) and TRITC-conjugated anti-goat IgG (1:250) (Sigma Chemical Co., St Louis, MO, USA) in the blocking buffer in darkness. After rinsing three times with PBS, the coverslips were mounted for fluorescent microscopy onto a drop of anti-fade mounting medium containing 50% glycerol, 100 mg/ml DABCO (1,4-diazabicyclo-[2.2.2.]octane, Sigma) and 0.05% sodium azide in PBS on microscope slides. Double-label immunofluorescent microscopy was performed using a laser scanning confocal microscope (Leica TCS 4 D, 100x /1.4 oil ICT:D objective, Heidelberg, Germany). With these immunocytochemical reactions, proNPY is stained as overlapping staining of green and red (becomes yellow), because the C-pon and NPY are co-localized. Red staining is a marker for sole NPY-ir and green staining as sole C-pon-ir.

### Results

### Baseline characteristics and Vo₂ₘₐₓ determinations

These were no differences in the mean baseline characteristics or VO₂ₘₐₓ values between study subjects in the two genotype groups (Table 1).

### NPY-genotype influences heart rate level

There was a statistically significant genotype effect in heart rate during the study period (Table 2) the Leu7Pro group having higher mean pulse rate over time (Fig. 1A). No statistically significant differences were however, detected in mean pulse rate at any separate time point between the groups. No differences were observed in systolic or diastolic blood pressure between the genotypes during the whole study period (Table 2, Fig. 1B).

### NPY-genotype influences exercise-induced increases in NPY and FFA concentrations

The Leu7Pro group had clearly higher overall plasma NPY concentration (Table 2, Fig. 2A) with statistically significant differences at 20 min maximal NPY concentrations (p < 0.05) and near significant differences at 30 min and 40 min post-exercise NPY concentrations (p = 0.05). The mean exercise-induced raise of NPY between 0 min and 20 min was 90.4 ± 12.7 pmol/L in the subjects with the Pro7 allele and 51.9 pmol/L ± 5.4 in subjects without this allele (p < 0.05, t test). There were no statistically significant differences in the concentrations of E and NE in plasma (Table 2, Fig. 2B and 2C) between the groups. The mean NE/NPY ratio in plasma was 84.9 ± 9.7 in Leu7Leu subjects and 56 ± 5.3 in Leu7Pro subjects (p < 0.05; t-test).

A dramatic difference was observed in the overall FFA concentrations between the genotype groups (Table 2, Fig. 3A); the Leu7Pro subjects had significantly lower FFA concentrations than Leu7Leu subjects, with the largest difference in post-exercise 40 min concentration (p < 0.05). NPY levels had a positive association with FFA concentrations in the Leu7Pro group (r = 0.45, p < 0.001) and in the Leu7Leu group (r = 0.36, p < 0.05). There were lower overall insulin concentrations in subjects with the Leu7Pro genotype and no clear exercise-induced reduction in insulin levels in this group (Table 2, Fig 3B), the statistically significant differences in concentrations were detected before the exercise at 0 min (p < 0.05) and after exercise at 60 min (p < 0.05). Lactate levels were identical in the two genotype groups throughout the study period (Table 2, Fig. 3C).

### NPY-genotype dertermines the pattern of NPY- and proNPY-ir in epithelial cells

Clear intracellular punctate staining (Fig. 4) could be seen in HWECs with immunocytohemical detection of proNPY and NPY. These clustered immunoreactive puncta were considered to represent peptides concentrated in some intracellular compartments of the cells. As shown in the Fig. 4, there was a marked difference in the pattern of staining between HUVECs of Leu7Pro and of Leu7Leu genotype, the former having prominent NPY-ir in addition to some proNPY-ir (Fig. 4B). In contrast, HUVECs with Leu7Leu genotype did not contain any NPY or C-pon staining, but exhibited profound vesicle-like structures with proNPY-ir (Fig. 4A).

### Discussion

Recent studies have linked the Leu7Pro polymorphism of preproNPY to risk factors and development of atherosclerosis in adults and in children²¹⁻²³. This polymorphism is likely to influence the intracellular processing of the synthesized preproNPY peptide as the mutation is located in the signal peptide part, which guides the newly-formed peptide into ER where it is cleaved off and the following further processing of proNPY leads to the formation of the mature secretory NPY. As a result of changed processing of the prohormone, the storage or kinetics of NPY release could be modified in subjects having this polymorphism. This hypothesis was tested in the current study, where NPY secretion was stimulated by high-intense cycle-ergometer exercise in healthy subjects having the Leu7Pro polymorphism and their matched Leu7Leu controls. Other responses reflecting the changes of sympathetic stimulation were also measured. In addition, human endothelial cells, which are known to contain and process NPY, were isolated, genotyped and studied by immunocytochemistry to view possible differences in proNPY and NPY contents of cells with the Pro7 substitution compared to cells without this substitution.

Marked increase in the excretion of NPY during exercise was detected in subjects with Leu7Pro genotype compared to controls. These subjects had also higher heart rate throughout the study period but no change in systolic or diastolic blood pressure. Furthermore, despite of similar exercise-induced catecholamine excretion and lower insulin concentrations, the subjects with the polymophism had clearly lower FFA release than the controls. Similar lactate levels between the groups indicated identical individual exercise intensity.

In plasma, NPY circulates mainly as an intact 36-aa peptide, but also as Y2-receptor ligand NPY₃₋₃₆, a product of dipeptidyl peptidase IV enzyme which is found in endothelium^{9,29}. NPY found in circulating blood in rest and during physical exercise is derived maily from perivascular symphatetic nerve-bundles^{16,20,30}. A minor proportion of plasma NPY level originates from endothelium ⁹. Although NPY and NE are co-released, NPY release is stimulated only in high-intensity exercise whereas NE is released more easily also in low-intensity exercise in healthy subjects^{19,31}. Therefore, 80% Vo₂ₘₐₓ level exrcise was used as a stimulator for symphathetic nervous system in this study.

The results show similar timing of exercise-induced NPY release in the two genotype groups but significantly higher NPY concentrations in subjects with the Leu7Pro genotype, the maximal values showing the largest difference compared to controls. There was no difference in the basal NPY concentrations but over 40 % higher rise from the basal to the maximal NPY concentration in the subjects with Pro7 allele. This suggests that more NPY is produced by the sympathetic nerves, NPY is more easily released by sympathetic stimulation or that the elimination of NPY is decreased in subjects with the polymorphism. The first two mechanisms seem more reasonable based on the known functions of signal peptides, and the similar elimination profile of NPY detected in this study. The increased stimulated NPY concentrations in Leu7Pro subjects may be also detected in tissue level, which has not been studied so far.

Catecholamine concentrations have not been previously compared between the subjects with and without the Pro7 allele. This study showed no differences between the genotypes in basal or exercise induced NE and E concentration. The mean NE/NPY ratio in plasma was 1.5 times smaller in the Leu7Pro genotype group compared to Leu7Leu group, which may reflect also different ratio of NE to NPY also in storage vesicles in symphatetic nerves and other cells.

The subjects with the NPY polymorphism had higher heart rate in the current study, the difference in the mean heart rate was 5 to 10 beats/min continuously during the study period, also during the 30 min pre-exercise resting period. This suggests that the NPY genotype regulates heart rate in healthy non-smoking subjects. As the catecholamine levels were not different between the study groups, the higher heart rate is not explained by their action. Although NPY is found in symphatetic nerve innervations in heart, where dense NPY-ir has been detected in close contact to nodal tissue and cardiac muscle fibres³², it is not known to have direct inotropic or chronotropic effects³³. However, NPY may promote parasymphatolytic cardiac responses by inhibiting acetylcholine release from colinergic nerves in heart ^{34,35}. that could result in higher pulse rate in the subjects with higher NPY release. The increased sympatovagal ratios found in type II diabetic patients with the Leu7Pro genotype²³ also support the conclusion that this genotype may have higher overall heart rate compared to Leu7Leu genotype.

Elevated NPY-concentrations in plasma have been reported in hypertension ³⁶⁻³⁸, although this has not been considered as the ethiology for the disease, but rather reflect sympathetic activation related to the disease. An earlier study with 966 middle-aged men indicated a slightly higher systolic (3 mmHg) and diastolic (2.1 mmHg) blood pressure in subjects with Pro7 substitution, but no difference in genotype frequencies in groups of hypertensive and normotensive subjects (Karvonen et al., unpublished observation). In the present study, no difference was detected in the diastolic or systolic blood pressure between the genotypes, maybe because of too small number of study subject to detect a comparable difference.

Despite of similar catecholamine levels, the two genotype groups had strikingly different exercise-induced FFA concentrations with the Leu7Pro group having clearly lower post-exercise values. Catecholamines rapidly promote lipolysis and raise the plasma FFA concentrations by increasing the rate of adipose tissue triacylglycerol mobilization by hormone-sensitive lipase. The increase in lipolysis during exercise is mainly mediated by adrenergic beta-receptor activation and the consequent increase in intracellular cAMP-concentration³⁹, which is known to be the main regulator of the activity of hormone-sensitive lipase. Consequently, hormones and drugs, including NPY that reduce intracellular cAMP consentrations in human adipocytes are able to inhibit lipolysis The inhibition of lipolysis in human adipocytes by NPY has been shown to be dose-dependent⁵ and therefore higher NPY release in Leu7Pro subjects during exercise may explain the difference in FFA concentration between the genotypes. The decrease in insulin concentrations during exercise have been shown to facilitate FFA mobilization during exercise⁴⁰⁻⁴². Therefore differences in plasma insulin concentrations observed between the groups do not explain the observed lower FFA levels in the Leu7Pro group since the insulin levels were lower in this group. Earlier studies have shown similar postprandial (Schwab et al., unpublished observation) or fasting insulin concentrations in Leu7Pro and Leu7Leu groups^{21,23}. Also, an earlier study have shown no differences in postprandial FFA levels or in lipoprotein lipase or hepatic lipase activity between the genotypes (Schwab et al., unpublished observation).

FFAs modulate the activity of cholesterol ester transfer protein (CEPT) by inhibiting the lipid transfer inhibitor protein (LTIP)⁴³. The more pronounced exercise-induced FFA-release in the Leu7Leu genotype may thus result in increased CEPT activity, which according to recent reports is atheroprotective⁴⁴. The FFA levels in plasma known to activate LTIP are 0.8-1.0 mmol/L⁴³. These levels are transiently produced by both genotypes at least in exercise but are more prolonged in the Leu7Leu genotype, thus leaving the Leu7Pro genotype subjects at higher risk for atherosclerosis.

The immunohistochemical studies of isolated and genotyped HWECs revealed clearly different picture of the NPY-related ir between the genotypes. With double-labelling of NPY and C-pon we could demonstrate that in human endothelial cells with Leu7Pro genotype the amount of NPY without C-pon was prominent. The endothelial cells with Leu7Leu genotype contained only NPY with C-pon, that is proNPY. This suggests difference in processing of the preproNPY between the genotypes. Accordingly, the Leu7Pro genotype cells seem to form mature NPY more efficiently, which suggests that the polymorphic signal peptide is functional and guides the nascent peptide readily into the intracellular processing and secretory pathways. Earlier studies have shown that NPY is a mitogenic substance, which clearly accelerates smooth muscle and endothelial cell proliferation ^{9,45,46}. The observed higher proportion ofNPY in HUVECs with the Leu7Pro genotype, may indicate higher local endothelial NPY release, and enhanced proliferation of the underlying intimal smooth muscle cell layer, which could be the mechanism for accelerated intima-media thickening observed in subjects with this genotype²³ (Karvonen et al., unpublished observation).

In conclusion, our study demonstrates significantly higher NPY concentrations in subjects with the Leu7Pro polymorphism compared to controls during exercise-induced sympathetic activation. These subjects also have increased overall heart rate and lower exercise-induced FFA-values, which may be secondary changes to increased NPY excretion. Studies with isolated HUVECs strengthens the functional consequence of the Pro7 substitution in the signal peptide of NPY leading to more efficient production of NPY. Earlier studies linked this polymorphism to increased levels of total cholesterol, LDL cholesterol and triglyserides in blood and to accelerated development of atherosclerosis²¹⁻²³, which may all be the ultimate consequences of increased NPY contents in blood and/or tissues of subjects with the Leu7Pro genotype.

It will be appreciated that the methods of the present invention can be incorporated in the form of a variety of embodiments, only a few of which are disclosed herein.

Thus, the described embodiments are illustrative and should not be construed as restrictive.

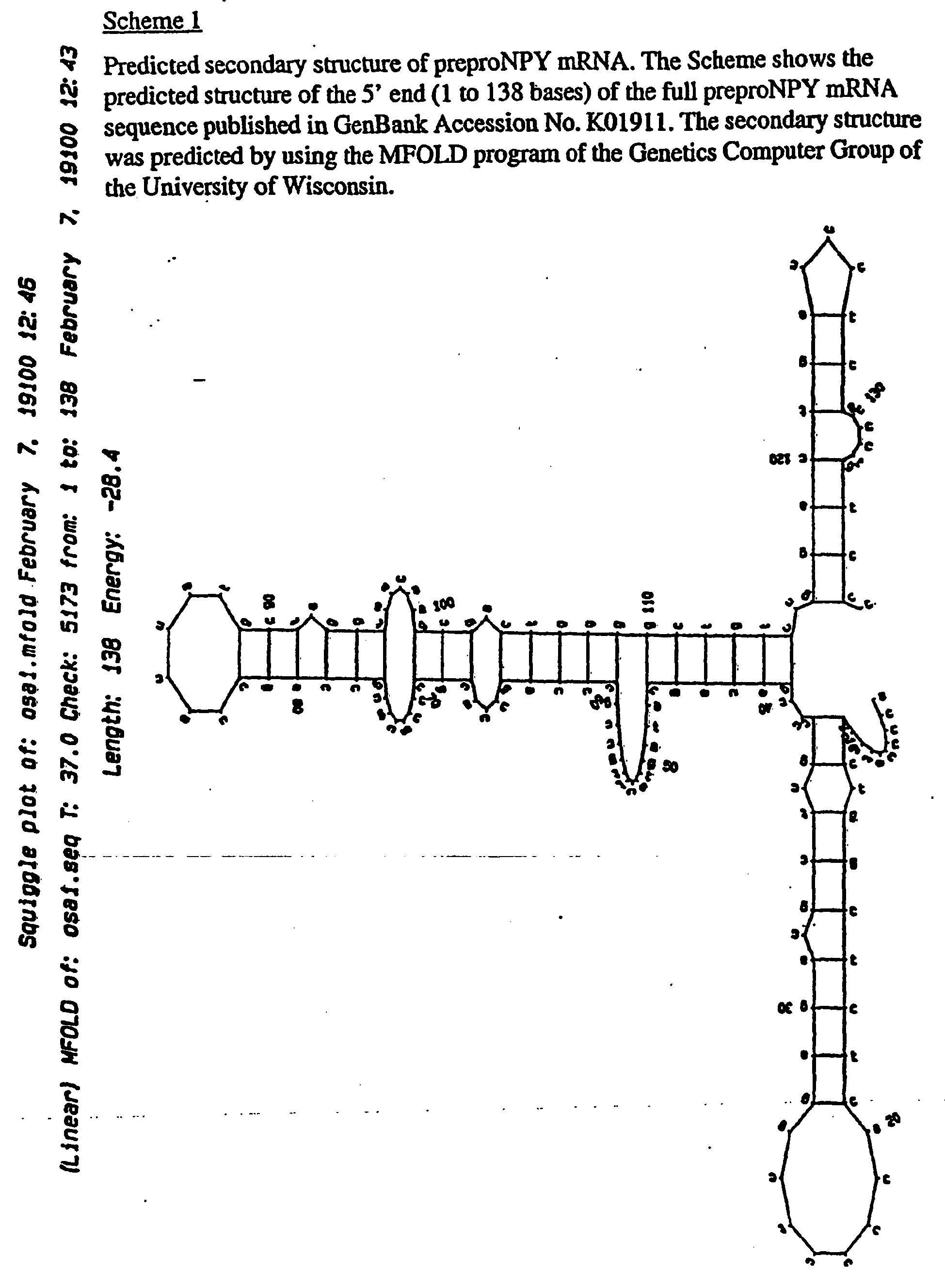

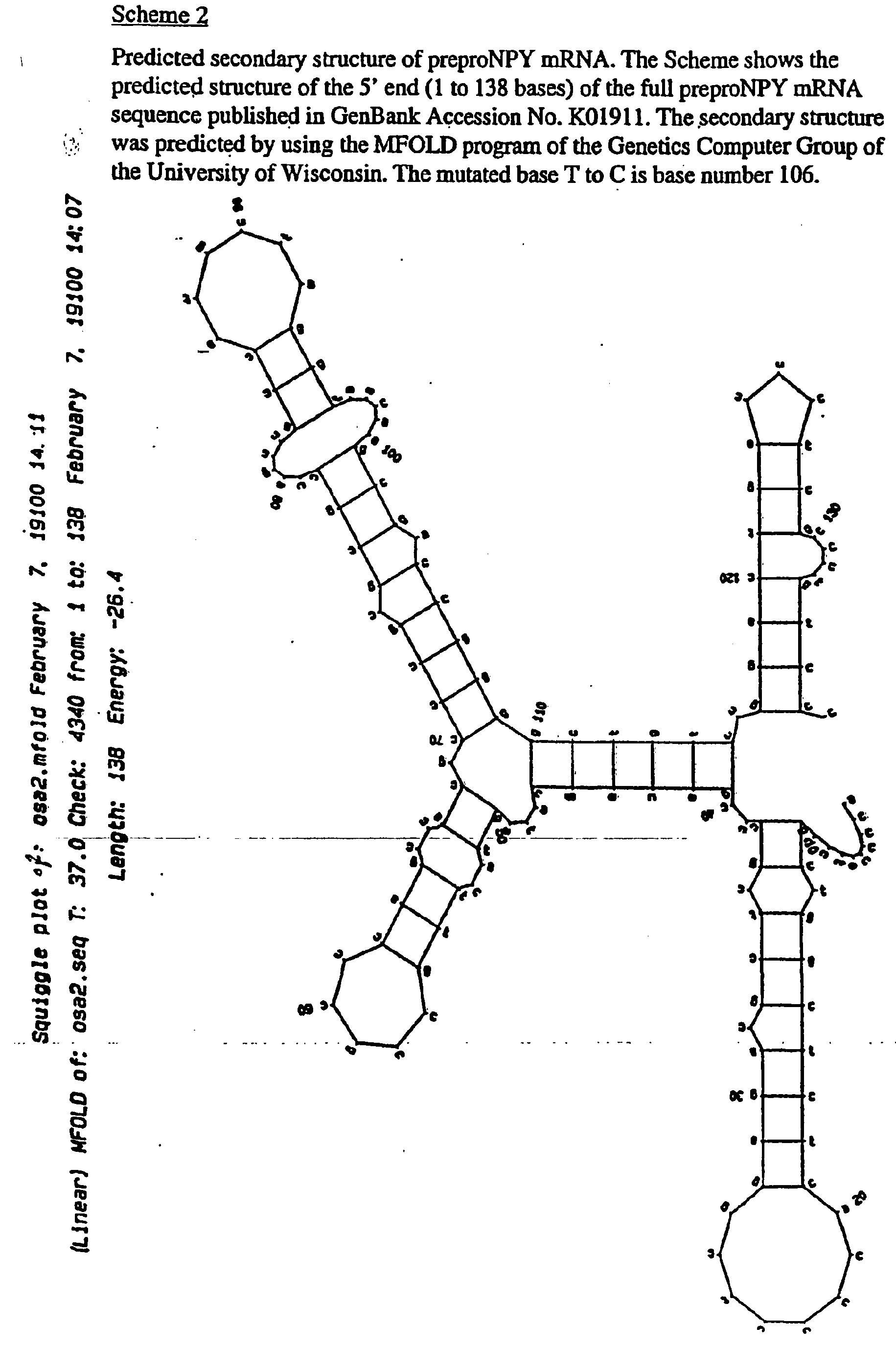

**Table 1. Baseline characteristics and VO_{2MAX} values (mean ± sem) were similar in the two genotype groups.**

| Leu7Leu genotype (n = 9) | | Leu7Pro genotype (n = 9) |
|---|---|---|
| No of males / females | 2 / 7 | 2 / 7 |
| BMI (kg/m²) | 22.8 ±0.9 | 22.0 ±0.8 |
| Body fat (%) | 23.6 ± 2.7 | 23.5 ± 2.4 |
| Age (y) | 22.1 ± 0.7 | 22.7 ±0.6 |
| Plasma glucose (mmol/L) | 4.6 ± 0.06 | 5.0 ± 0.03 |
| Serum cholesterol (mmol/L) | 4.8 ± 0.08 | 4.4 ± 0.07 |
| Serum LDL cholesterol (mmol/L) | 2.8 ± 0.06 | 2.2 ± 0.09 |
| Vo₂ₘₐₓ (ml/kg/min) | 47.7 ± 2.4 | 42.9 ± 2.7 |

**Table 2. Effects of genotype, time and genotype x time on the measured parameters (repeated measures ANOVA for mixed models).**

| | Genotype | | Time | | Genotype x Time | |
|---|---|---|---|---|---|---|
| | F-value | *P*-value | F- value | P- value | F- value | P- value |
| Heart rate | 5.47 | 0.03 | 244.5 | 0.0001 | 0.24 | 0.99 |
| Systolic blood pressure | 0.13 | 0.72 | 11.7 | 0.0001 | 1.44 | 0.18 |
| Diastolic blood pressure | 0.92 | 0.35 | 1.5 | 0.15 | 0.64 | 0.76 |
| Neuropeptide Y | 4.17 | 0.058 | 36.5 | 0.0001 | 2.42 | 0.018 |
| Norepinephrine | 0.11 | 0.74 | 178.2 | 0.0001 | 0.67 | 0.72 |
| Epinephrine ' | 0.01 | 0.92 | 68.2 | 0.0001 | 1.91 | 0.064 |
| Free fatty acids | 0.62 | 0.44 | 24.5 | 0.0001 | 2.21 | 0.03 |
| Insulin | 1.43 | 0.25 | 4.83 | 0.0001 | 2.72 | 0.008 |
| Lactate | 0.1 | 0.76 | 143.5 | 0.0001 | 0.54 | 0.82 |

### REFERENCES

1. Blomqvist AG, Herzog H. Y-receptor subtypes--how many more? Trends Neurosci 1997; 20:294-298.
2. Gerald C, Walker MW, Criscione L, Gustafson EL, Batzl-Hartmann C, Smith KE, et al. A receptor subtype involved in neuropeptide-Y-induced food intake [see comments]. Nature 1996; 382:168-171.
3. Zukowska-Grojec Z. Neuropeptide Y. A novel sympathetic stress hormone and more. Ann N Y Acad Sci 1995; 771:219-233.
4. Castan I, Valet P, Quideau N, Voisin T, Ambid L, Laburthe M, et al. Antilipolytic effects of alpha 2-adrenergic agonists, neuropeptide Y, adenosine, and PGE1 in mammal adipocytes. Am J Physiol 1994; 266:R1141-R1 147
5. Valet P, Berlan M, Beauville M, Crampes F, Montastruc JL, Lafontan M. Neuropeptide Y and peptide YY inhibit lipolysis in human and dog fat cells through a pertussis toxin-sensitive G protein. J Clin Invest 1990; 85:291-295.
6. Bischoff A, Michel MC. Renal effects of neuropeptide Y. Pflugers Arch 1998; 435:443-4.53.
7. Morgan DG, Kulkarni RN, Hurley JD, Wang ZL, Wang RM, Ghatei MA, et al. Inhibition of glucose stimulated insulin secretion by neuropeptide Y is mediated via the Y1 receptor and inhibition of adenylyl cyclase in RIN 5AH rat insulinoma cells. Diabetologia 1998; 41:1482-1491.
8. Kalra SP, Sahu A, Kalra PS, Crowley WR. Hypothalamic neuropeptide Y: a circuit in the regulation of gonadotropin secretion and feeding behavior. Ann N Y Acad Sci 1990; 611:273-283.
9. Zukowska-Grojec Z, Karwatowska-Prokopczuk E, Rose W, Rone J, Movafagh S, Ji H, et al. Neuropeptide Y: a novel angiogenic factor from the sympathetic nerves and endothelium. Circ Res 1998; 83:187-195.
10. Tatemoto K, Carlquist M, Mutt V. Neuropeptide Y--a novel brain peptide with structural similarities to peptide YY and pancreatic polypeptide. Nature 1982; 296:659-660.
11. Edvinsson L, Emson P, McCulloch J, Tatemoto K, Uddman R. Neuropeptide Y: cerebrovascular innervation and vasomotor effects in the cat. Neurosci Lett 1983; 43:79-84.
12. Edvinsson L, Ekblad E, Hakanson R, Wahlestedt C. Neuropeptide Y potentiates the effect of various vasoconstrictor agents on rabbit blood vessels. Br J Pharmacol 1984; 83:519-525.
13. Ekblad E, Edvinsson L, Wahlestedt C, Uddman R, Hakanson R, Sundler F. Neuropeptide Y co-exists and co-operates with noradrenaline in perivascular nerve fibers. Regul Pept 1984; 8:225-235.
14. Fried G, Terenius L, Brodin E, Efendic S, Dockray G, Fahrenkrug J, et al. Neuropeptide Y, enkephalin and noradrenaline coexist in sympathetic neurons innervating the bovine spleen. Biochemical and immunohistochemical evidence. Cell Tissue Res 1986; 243:495-508.
15. Lundberg JM, Hemsen A, Fried G, Theodorsson-Norheim E, Lagercrantz H. Co-release of neuropeptide Y (NPY)-like immunoreactivity and catecholamines in newborn infants. Acta Physiol Scand 1986; 126:471-473.
16. Lundberg JM, Martinsson A, Hemsen A, Theodorsson-Norheim E, Svedenhag J, Ekblom B, et al. Co-release of neuropeptide Y and catecholamines during physical exercise in man. Biochem Biophys Res Commun 1985; 133:30-36.
17. Dahlof C, Dahlof P, Lundberg JM, Strombom U. Elevated plasma concentration of neuropeptide Y and low level of circulating adrenaline in elderly asthmatics during rest and acute severe asthma. Pulm Pharmacol 1988; 1:3-6.
18. Miller MA, Sagnella GA, Markandu ND, MacGregor GA. Radioimmunoassay for plasma neuropeptide-Y in physiological and physiopathological states and response to sympathetic activation. Clin Chim Acta 1990; 192:47-53.
19. Morris MJ, Russell AE, Kapoor V, Cain MD, Elliott JM, West MJ, et al. Increases in plasma neuropeptide Y concentrations during sympathetic activation in man. J Auton Nerv Syst 1986; 17:143-149.
20. Pernow J, Lundberg JM, Kaijser L, Hjemdahl P, Theodorsson-Norheim E, Martinsson A, et al. Plasma neuropeptide Y-like immunoreactivity and catecholamines during various degrees of sympathetic activation in man. Clin Physiol 1986; 6:561-578.
21. Karvonen MK, Pesonen U, Koulu M, Niskanen L, Laakso M, Rissanen A, et al. Association of a leucine(7)-to-proline(7) polymorphism in the signal peptide of neuropeptide Y with high serum cholesterol and LDL cholesterol levels. Nat Med 1998; 4:1434-1437.
22. Karvonen MK, Koulu M, Pesonen U, Uusitupa MI, Tammi A, Viikari J, et al. Leucine 7 to proline 7 polymorphism in the preproneuropeptide Y is associated with birth weight and serum triglyceride concentration in preschool aged children. J Clin Endocrinol Metab 2000; 85:1455-1460.
23. Niskanen L, Karvonen MK, Valve R, Koulu M, Pesonen U, Mercuri M, et al. Leucine 7 to proline 7 polymorphism in the neuropeptide Y gene is associated with enhanced carotid atherosclerosis in elderly patients with type 2 diabetes and control subjects [In Process Citation]. J Clin Endocrinol Metab 2000; 85:2266-2269.
24. Paquet L, Massie B, Mains RE. Proneuropeptide Y processing in large dense-core vesicles: manipulation of prohormone convertase expression in sympathetic neurons using adenoviruses. J Neurosci 1996; 16:964-973.
25. Ryan P, Edwards CO. Systematic introduction of proline in a eukaryotic signal sequence suggests asymmetry within the hydrophobic core. J Biol Chem 1995; 270:27876-27879.
26. Fitches AC, Appleby R, Lane DA, De S, V, Leone G, Olds RJ. Impaired cotranslational processing as a mechanism for type I antithrombin deficiency. Blood 1998; 92:4671-4676.
27. Durnin JV, Womersley J. Body fat assessed from total body density and its estimation from skinfold thickness: measurements on 481 men and women aged from 16 to 72 years. Br J Nutr 1974; 32:77-97.
28. Scheinin M, Karhuvaara S, Ojala-Karlsson P, Kallio A, Koulu M. Plasma 3,4-dihydroxyphenylglycol (DHPG) and 3-methoxy-4-hydroxyphenylglycol (MHPG) are insensitive indicators of alpha 2-adrenoceptor mediated regulation of norepinephrine release in healthy human volunteers. Life Sci 1991; 49:75-84.
29. Mentlein R, Dahms P, Grandt D, Kruger R. Proteolytic processing of neuropeptide Y and peptide YY by dipeptidyl peptidase IV. Regul Pept 1993; 49:133-144.
30. Bernet F, Dedieu JF, Laborie C, Montel V, Dupouy JP. Circulating neuropeptide Y (NPY) and catecholamines in rat under resting and stress conditions. Arguments for extra-adrenal origin of NPY, adrenal and extra-adrenal sources of catecholamines. Neurosci Lett 1998; 250:45-48.
31. Takiyyuddin MA, Brown MR, Dinh TQ, Cervenka JH, Braun SD, Parmer RJ, et al. Sympatho-adrenal secretion in humans: factors governing catecholamine and storage vesicle peptide co-release. J Auton Pharmacol 1994; 14:187-200.
32. Gu J, Polak JM, Adrian TE, Allen JM, Tatemoto K, Bloom SR. Neuropeptide tyrosine (NPY)--a major cardiac neuropeptide. Lancet 1983; 1:1008-1010.
33. Allen JM, Gjorstrup P, Bjorkman JA, Ek L, Abrahamsson T, Bloom SR. Studies on cardiac distribution and function of neuropeptide Y. Acta Physiol Scand 1986; 126:405-411.
34. Potter E. Presynaptic inhibition of cardiac vagal postganglionic nerves by neuropeptide Y. Neurosci Lett 1987; 83:101-106.
35. Potter EK. Cardiac vagal action and plasma levels of neuropeptide Y following intravenous injection in the dog. Neurosci Lett 1987; 77:243-247.
36. Chalmers J, Morris M, Kapoor V, Cain M, Elliott J, Russell A, et al. Neuropeptide Y in the sympathetic control of blood pressure in hypertensive subjects. Clin Exp Hypertens [A] 1989; 11 Suppl 1:59-66.
37. Solt VB, Brown MR, Kennedy B, Kolterman OG, Ziegler MG. Elevated insulin, norepinephrine, and neuropeptide Y in hypertension. Am J Hypertens 1990; 3:823-828.
38. Lettgen B, Wagner S, Hanze J, Lang RE, Rascher W. Elevated plasma concentration of neuropeptide Y in adolescents with primary hypertension. J Hum Hypertens 1994; 8:345-349.
39. Arner P, Kriegholm E, Engfeldt P, Bolinder J. Adrenergic regulation of lipolysis in situ at rest and during exercise. J Clin Invest 1990; 85:893-898.
40. Hirsch IB, Marker JC, Smith LJ, Spina RJ, Parvin CA, Holloszy JO, et al. Insulin and glucagon in prevention of hypoglycemia during exercise in humans. Am J Physiol 1991; 260:E695-E704
41. Wasserman DH, Lacy DB, Goldstein RE, Williams PE, Cherrington AD. Exercise-induced fall in insulin and increase in fat metabolism during prolonged muscular work. Diabetes 1989; 38:484-4.90.
42. Wasserman DH, Williams PE, Lacy DB, Goldstein RE, Cherrington AD. Exercise-induced fall in insulin and hepatic carbohydrate metabolism during muscular work. Am J Physiol 1989; 256:E500-E509
43. Morton RE, Greene DJ. Suppression of lipid transfer inhibitor protein activity by oleate. A novel mechanism of cholesteryl ester transfer protein regulation by plasma free fatty acids. Arterioscler Thromb Vasc Biol 1997; 17:3041-3048.
44. Stein O, Stein Y. Atheroprotective mechanisms of HDL. Atherosclerosis 1999; 144:285-301.
45. Nilsson T, Edvinsson L. Neuropeptide Y stimulates DNA synthesis in human vascular smooth muscle cells through neuropeptide Y Y1 receptors. Can J Physiol Pharmacol 2000; 78:256-259.
46. Zukowska-Grojec Z, Pruszczyk P, Colton C, Yao J, Shen GH, Myers AK, et al. Mitogenic effect of neuropeptide Y in rat vascular smooth muscle cells. Peptides 1993; 14:263-268.

## Claims

1. The use of a substance for the manufacture of a pharmaceutical composition for use in a method for reducing stress-induced overproduction of neuropeptide Y (NPY) in an individual, wherein the stress-induced overproduction of NPY is caused by a polymorphism comprising the substitution of the position 7 leucine for proline in the signal peptide part of the human preproNPY, wherein
- said method is aimed to modulate an overactive NPY system in said individual, or
- said individual is subjected to a method aimed to reduce or prevent expression of the mutated allele causing said polymorphism, and said substance is
- a Y1-receptor antagonist selected from the group consisting of BIBO 3304; BIBP 3226; GR231118; and a 1,3-disubstituted benzazepine,
- a Y2-receptor antagonist which is BIIE0246,
- a Y5-receptor antagonist selected from the group consisting of L-152,804 and CGP71683A,
- an NPY-antibody,
- an antisense oligonucleotide,
- a peptide nucleic acid (PNA), or
- a ribozyme.

2. The use according to claim 1 wherein said method is used to reduce constitutive overexpression of NPY in endothelial cells of said individual.

3. The use according to claim 1 wherein said method is used to reduce heart rate in said individual.

4. The use according to claim 1 wherein said method is used to enhance insulin secretion in said individual.

5. The use according to any of the claims 1 to 4 wherein an overproduction of NPY is counteracted by administering an antagonist as defined in claim 1 to said individual.

6. The use according to claim 5 wherein said antagonist is aimed to decrease the expression of the NPY gene.

7. The use according to claim 5 wherein said antagonist is an NPY receptor antagonist as defined in claim 1.

8. The use according to claim 5 wherein said antagonist is an NPY antibody.

9. The use according to claim 8 wherein said antibody is an antibody reacting with the NPY in serum.

10. The use according to any of the claims 1-4 wherein said method is a specific gene therapy aimed to repair the mutated allele.

11. The use according to claim 10 which comprises the use of an antisense oligonucleotide.

12. The use according to claim 10 which comprises the use of a peptide nucleid acid (PNA).

13. The use according to claim 10 which comprises the use of a ribozyme.

14. A method,for diagnosing a person's susceptibility for having a risk for stress-induced overproduction of neuropeptide Y (NPY), said method comprising determining in vitro whether said subject has a polymorphism in the signal peptide part of the human preproNPY, said polymorphism comprising the subsitution of the position 7 leucine for proline in the signal peptide part of said preproNPY, said polymorphism being indicative of a risk for stress-induced overproduction of NPY.

## Patentansprüche

1. Verwendung einer Substanz für die Herstellung einer pharmazeutischen Zusammensetzung zur Verwendung in einem Verfahren zum Verringern der Stress-induzierten Überproduktion des Neuropeptides Y (NPY) bei einem Individuum, wobei die Stress-induzierte Überproduktion von NPY durch einen Polymorphismus, umfassend die Substitution des Position 7-Leucins durch Prolin in dem Signalpeptidteil von humanem Präpro-NPY, verursacht wird, wobei
- das Verfahren darauf abzielt, ein überaktives NPY-System in dem Individuum zu modulieren oder
- das Individuum einem Verfahren unterzogen wird, das darauf abzielt, die Expression des mutierten Allels, das den Polymorphismus verursacht, zu verringern oder zu verhindern, und die Substanz
- ein Y1-Rezeptorantagonist, ausgewählt aus der Gruppe, bestehend aus BIBO 3304, BIBP 3226, GR231118 und einem 1,3-disubstituierten Benzazepin,
- ein Y2-Rezeptorantagonist, der BIIE0246 ist,
- ein Y5-Rezeptorantagonist, ausgewählt aus der Gruppe, bestehend aus L-152,804 und CGP71683A,
- ein NPY-Antikörper
- ein Antisense-Oligonucleotid,
- eine Peptidnucleinsäure (PNA) oder
- ein Ribozym ist.

2. Verwendung gemäß Anspruch 1, wobei das Verfahren verwendet wird, um die konstitutive Überexpression von NPY in Endothelzellen des Individuums zu verringern.

3. Verwendung gemäß Anspruch 1, wobei das Verfahren verwendet wird, um die Herzfrequenz in dem Individuum zu verringern.

4. Verwendung gemäß Anspruch 1, wobei das Verfahren verwendet wird, um die Insulinsekretion in dem Individuum zu verstärken.

5. Verwendung gemäß einem der Ansprüche 1 bis 4, wobei einer Überexpression von NPY durch Verabreichen eines Antagonisten, wie in Anspruch 1 definiert, an das Individuum entgegengewirkt wird.

6. Verwendung gemäß Anspruch 5, wobei der Antagonist auf die Verringerung der Expression des NPY-Gens abzielt.

7. Verwendung gemäß Anspruch 5, wobei der Antagonist ein NPY-Rezeptorantagonist, wie in Anspruch 1 definiert, ist.

8. Verwendung gemäß Anspruch 5, wobei der Antagonist ein NPY-Antikörper ist.

9. Verwendung gemäß Anspruch 8, wobei der Antikörper ein Antikörper ist, der mit dem NPY in Serum reagiert.

10. Verwendung gemäß einem der Ansprüche 1 bis 4, wobei das Verfahren eine spezifische Gentherapie ist, die darauf abzielt, das mutierte Allel zu reparieren.

11. Verwendung gemäß Anspruch 10, die die Verwendung eines Antisense-Oligonucleotids umfasst.

12. Verwendung gemäß Anspruch 10, die die Verwendung einer Peptidnucleinsäure (PNA) umfasst.

13. Verwendung gemäß Anspruch 10, die die Verwendung eines Ribozyms umfasst.

14. Verfahren zum Diagnostizieren der Anfälligkeit einer Person dafür, ein Risiko für Stress-induzierte Überproduktion des Neuropeptids Y (NPY) zu haben, wobei das Verfahren das Bestimmen in vitro umfasst, ob die Person einen Polymorphismus in dem Signalpeptidteil von humanem Präpro-NPY hat, wobei der Polymorphismus die Substitution des Position 7-Leucins durch Prolin in dem Signalpeptidteil von humanem Präpro-NPY umfasst, wobei der Polymorphismus für ein Risiko für Stress-induzierte Überproduktion von NPY hinweisend ist.

## Revendications

1. Utilisation d'une substance pour la fabrication d'une composition pharmaceutique à utiliser dans un procédé de réduction d'une surproduction de neuropeptide Y (NPY) induite par le stress chez un individu, dans laquelle la surproduction de NPY induite par le stress est due à un polymorphisme comprenant la substitution de la proline en position 7 par la leucine dans la partie peptide signal du précurseur de NPY humain, dans laquelle
- ledit procédé est destiné à moduler un système de NPY hyperactif chez ledit individu, ou
- ledit individu est soumis à un procédé destiné à réduire ou à empêcher l'expression de l'allèle muté entraînant ledit polymorphisme, et ladite substance est
- un antagoniste du récepteur Y1 choisi dans le groupe constitué par BIBO 3304 ; BIBP 3226 ; GR231118 ; et une benzazépine 1,3-disubstituée,
- un antagoniste du récepteur Y2 qui est BIIE0246,
- un antagoniste du récepteur Y5 choisi dans le groupe constitué par L-152 804 et CGP71683A,
- un anticorps anti-NPY,
- un oligonucléotide anti-sens,
- un acide peptido-nucléique (PNA), ou
- un ribozyme.

2. Utilisation selon la revendication 1, dans laquelle ledit procédé est utilisé pour réduire la surexpression constitutive du NPY dans les cellules endothéliales dudit individu.

3. Utilisation selon la revendication 1, dans laquelle ledit procédé est utilisé pour faire baisser le rythme cardiaque chez ledit individu.

4. Utilisation selon la revendication 1, dans laquelle ledit procédé est utilisé pour renforcer la sécrétion d'insuline chez ledit individu.

5. Utilisation selon l'une quelconque des revendications 1 à 4, dans laquelle une surproduction de NPY est neutralisée par l'administration d'un antagoniste tel que défini dans la revendication 1 au dit individu.

6. Utilisation selon la revendication 5, dans laquelle ledit antagoniste est destiné à réduire l'expression du gène du NPY.

7. Utilisation selon la revendication 5, dans laquelle ledit antagoniste est un antagoniste du récepteur du NPY tel que défini dans la revendication 1.

8. Utilisation selon la revendication 5, dans laquelle ledit antagoniste est un anticorps anti-NPY.

9. Utilisation selon la revendication 8, dans laquelle ledit anticorps est un anticorps réagissant avec le NPY dans le sérum.

10. Utilisation selon l'une quelconque des revendications 1 à 4, dans laquelle ledit procédé est une thérapie génique spécifique destinée à réparer l'allèle muté.

11. Utilisation selon la revendication 10, qui comprend l'utilisation d'un oligonucléotide anti-sens.

12. Utilisation selon la revendication 10, qui comprend l'utilisation d'un acide peptido-nucléique (PNA).

13. Utilisation selon la revendication 10, qui comprend l'utilisation d'un ribozyme.

14. Procédé de diagnostic de la prédisposition d'une personne à un risque de surproduction de neuropeptide Y (NPY) induite par le stress, ledit procédé comprenant l'étape consistant à déterminer *in vitro* si ledit sujet présente un polymorphisme dans la partie peptide signal du précurseur de NPY humain, ledit polymorphisme comprenant la substitution de la proline en position 7 par la leucine dans la partie peptide signal dudit précurseur de NPY, ledit polymorphisme étant indicateur d'un risque de surproduction de NPY induite par le stress.
